# EUROPEAN PATENT SPECIFICATION

(11) **EP 2 804 877 B1**
(45) Date of publication and mention of the grant of the patent: **22.08.2018**
(21) Application number: 13701014.6
(22) Date of filing: 17.01.2013
(51) Int. Cl.: C07K 14/56, C07K 16/28, C07K 16/32, C07K 19/00

(54) **TARGETED MUTANT ALPHA-HELICAL BUNDLE CYTOKINES**
GEZIELTE MUTANTE ALPHA-HELIKALE BÜNDELCYTOKINE
CYTOKINES EN FAISCEAU À HÉLICES ALPHA MUTANTES À CIBLE SPÉCIFIQUE

(30) Priority: 20.01.2012 EP 12305075
(43) Date of publication of application: 26.11.2014
(73) Proprietor: VIB vzw, 9052 Ghent (BE); Universiteit Gent, 9000 Gent (BE); Centre National de la Recherche Scientifique, 75794 Paris Cedex 16 (FR); Université de Montpellier, 34090 Montpellier (FR); Centre Hospitalier Régional Universitaire de Montpellier, 34295 Montpellier Cedex 5 (FR); Universität Osnabrück, 49074 Osnabrück (DE)
(72) Inventor: TAVERNIER, Jan, B-9860 Balegem (BE); UZÉ, Gilles, F-34090 Montpellier (FR); CARTRON, Guillaume, F-34980 Combaillaux (FR); PAUL, Franciane, F-34090 Montpellier (FR); PIEHLER, Jacob, 49078 Osnabrück (DE)
(74) Representative: Grund, Martin
(86) International application number: PCT/EP2013/050787
(87) International publication number: WO 2013/107791

(56) References cited:
- WO-A1-2009/039409
- WO-A2-2008/124086
- WO-A2-2013/059885
- MASCI P ET AL: "NEW AND MOFIFIED INTERFERON ALFAS: PRECLINICAL AND CLINICAL DATA", CURRENT ONCOLOGY REPORTS, vol. 5, no. 2, 1 March 2003 (2003-03-01), pages 108-113, XP008037765, CURRENT SCIENCE, GB ISSN: 1523-3790
- ROISMAN L C ET AL: "Structure of the interferon-receptor complex determined by distance constraints from double-mutant cycles and flexible docking", PROCEEDINGS OF THE NATIONAL ACADEMY OF SCIENCES, vol. 98, no. 23, 6 November 2001 (2001-11-06), pages 13231-13236, XP002242615, US ISSN: 0027-8424, DOI: 10.1073/PNAS.221290398
- COULSTOCK EDWARD ET AL: "Liver-targeting of interferon-alpha with tissue-specific domain antibodies.", PLOS ONE, vol. 8, no. 2, E57263, February 2013 (2013-02), pages 1-11, XP002697904, ISSN: 1932-6203

### SEQUENCE LISTING

<110> VIB VZW
   UNIVERSITEIT GENT
   CENTRE NATIONAL DE LA RECHERCHE SCIENTIFIQUE
   UNIVERSITÉ MONTPELLIER 2
   CENTRE HOSPITALIER REGIONAL UNIVERSITAIRE DE MONTPELLIER UNIVERSITÄT OSNABRÜCK
<120> TARGETED MUTANT ALPHA-HELICAL BUNDLE CYTOKINES
<130> JT/TARG/403
<150> EP 12305075.9
   <151> 2012-01-20
<160> 10
<170> PatentIn version 3.5
<210> 1
   <211> 108
   <212> DNA
   <213> Artificial Sequence
<220>
   <223> Primer
<400> 1
   ggggggtccg gaccatcacc atcaccatca ccatcaccat caccctgctt ctcccgcctc       60
   cccagcatca cctgccagcc cagcaagtga tagcctggaa tttattgc                  108
<210> 2
   <211> 28
   <212> DNA
   <213> Artificial Sequence
<220>
   <223> Primer
<400> 2
   cgtctagatc attccttact tcttaaac 28
<210> 3
   <211> 21
   <212> DNA
   <213> Artificial Sequence
<220>
   <223> Primer
<400> 3
   cacgactgta ggccccagcg a 21
<210> 4
   <211> 21
   <212> DNA
   <213> Artificial Sequence
<220>
   <223> Primer
<400> 4
   agcagctgtc tctcccctcc g 21
<210> 5
   <211> 20
   <212> DNA
   <213> Artificial Sequence
<220>
   <223> Primer
<400> 5
   agagggaaat cgtgcgtgac 20
<210> 6
   <211> 21
   <212> DNA
   <213> Artificial Sequence
<220>
   <223> Primer
<400> 6
   caatagtgat gacctggccg t 21
<210> 7
   <211> 20
   <212> DNA
   <213> Artificial Sequence
<220>
   <223> Primer
<400> 7
   gagctagagc ctgcagcaat 20
<210> 8
   <211> 20
   <212> DNA
   <213> Artificial Sequence
<220>
   <223> Primer
<400> 8
   ttctgggcaa tctgcttctt 20
<210> 9
   <211> 352
   <212> PRT
   <213> Artificial Sequence
<220>
   <223> H6-Leptin construct
<220>
   <221> MISC_FEATURE
   <222> (94)..(94)
   <223> Xaa can be Ser or Asn
<400> 9
   His His His His His His Gly Gly Ser Gly Ile Gln Lys Val Gln Asp
   1               5                   10                  15
   Asp Thr Lys Thr Leu Ile Lys Thr Ile Val Thr Arg Ile Asn Asp Ile
               20                  25                  30
   Ser His Thr Gln Ser Val Ser Ala Lys Gln Arg Val Thr Gly Leu Asp
           35                  40                  45
   Phe Ile Pro Gly Leu His Pro Ile Leu Ser Leu Ser Lys Met Asp Gln
       50                  55                  60
   Thr Leu Ala Val Tyr Gln Gln Val Leu Thr Ser Leu Pro Ser Gln Asn
   65                  70                  75                  80
   Val Leu Gln Ile Ala Asn Asp Leu Glu Asn Leu Arg Asp Xaa Leu His
                   85                  90                  95
   Leu Leu Ala Phe Ser Lys Ser Cys Ser Leu Pro Gln Thr Ser Gly Leu
               100                 105                 110
   Gln Lys Pro Glu Ser Leu Asp Gly Val Leu Glu Ala Ser Leu Tyr Ser
           115                 120                 125
   Thr Glu Val Val Ala Leu Ser Arg Leu Gln Gly Ser Leu Gln Asp Ile
       130                 135                 140
   Leu Gln Gln Leu Asp Val Ser Pro Glu Cys Ala Leu Asp Gly Gly Ser
   145                 150                 155                 160
   Gly Gly Ser Gly Gly Ser Gly Gly Ser Gly Gly Ser Gly Gly Ser Gly
                   165                 170                 175
   Gly Ser Gly Gly Ser Gly Gly Ser Gly Gly Ser Gly Gly Ser Gly Gly
               180                 185                 190
   Ser Gly Gly Ser Gly Gly Ser Gly Gly Ser Gly Gly Ser Gly Gly Ser
           195                 200                 205
   Gly Gly Ser Gly Gly Ser Gly Gly Ser Ser Gly Ser Met Ala Gln Val
       210                 215                 220
   Gln Leu Gln Glu Ser Gly Gly Gly Val Val Gln Pro Gly Gly Ser Leu
   225 230 235 240
   Thr Leu Ser Cys Thr Arg Thr Gly Phe Thr Ala Ser Thr Asn Ala Val
   245 250 255
   Gly Trp Tyr Arg Gln Gly Pro Gly Lys Lys Cys Glu Trp Val Ser Tyr
   260 265 270
   Met Thr Ile Pro Ser Gly Arg Thr Thr Tyr Ala Asp Ala Val Lys Gly
   275 280 285
   Arg Phe Ala Met Ser Arg Asp Lys Ala Lys Ser Thr Val Phe Leu Gln
   290 295 300
   Met Asn Ser Leu Lys Pro Glu Asp Thr Ala Val Tyr Tyr Cys Gly Asp
   305 310 315 320
   Val Pro Phe Ser Thr Leu Pro Ala Met Cys Thr Asn Asp Gly Pro Trp
   325 330 335
   Gly Gln Gly Thr Gln Val Thr Val Ser Ser His His His His His His
   340 345 350
<210> 10
   <211> 345
   <212> PRT
   <213> Artificial Sequence
<220>
   <223> Mleptin construct
<220>
   <221> MISC_FEATURE
   <222> (88)..(88)
   <223> Xaa can be Ser or Asn
<400> 10
   Gly Gly Ser Gly Ile Gln Lys Val Gln Asp Asp Thr Lys Thr Leu Ile
   1               5                   10                  15
   Lys Thr Ile Val Thr Arg Ile Asn Asp Ile Ser His Thr Gln Ser Val
               20                  25                  30
   Ser Ala Lys Gln Arg Val Thr Gly Leu Asp Phe Ile Pro Gly Leu His
           35                  40                  45
   Pro Ile Leu Ser Leu Ser Lys Met Asp Gln Thr Leu Ala Val Tyr Gln
       50                  55                  60
   Gln Val Leu Thr Ser Leu Pro Ser Gln Asn Val Leu Gln Ile Ala Asn
   65                  70                  75                  80
   Asp Leu Glu Asn Leu Arg Asp Xaa Leu His Leu Leu Ala Phe Ser Lys
                   85                  90                  95
   Ser Cys Ser Leu Pro Gln Thr Ser Gly Leu Gln Lys Pro Glu Ser Leu
               100                 105                 110
   Asp Gly Val Leu Glu Ala Ser Leu Tyr Ser Thr Glu Val Val Ala Leu
           115                 120                 125
   Ser Arg Leu Gln Gly Ser Leu Gln Asp Ile Leu Gln Gln Leu Asp Val
       130                 135                 140
   Ser Pro Glu Cys Ala Leu Asp Gly Gly Ser Gly Gly Ser Gly Gly Ser
   145                 150                 155                 160
   Gly Gly Ser Gly Gly Ser Gly Gly Ser Gly Gly Ser Gly Gly Ser Gly
                   165                 170                 175
   Gly Ser Gly Gly Ser Gly Gly Ser Gly Gly Ser Gly Gly Ser Gly Gly
               180                 185                 190
   Ser Gly Gly Ser Gly Gly Ser Gly Gly Ser Gly Gly Ser Gly Gly Ser
           195                 200                 205
   Gly Gly Ser Ser Gly Ser Met Ala Gln Val Gln Leu Gln Glu Ser Gly
       210                 215                 220
   Gly Gly Val Val Gln Pro Gly Gly Ser Leu Thr Leu Ser Cys Thr Arg
   225                 230                 235                 240
   Thr Gly Phe Thr Ala Ser Thr Asn Ala Val Gly Trp Tyr Arg Gln Gly
                   245                 250                 255
   Pro Gly Lys Lys Cys Glu Trp Val Ser Tyr Met Thr Ile Pro Ser Gly
               260                 265                 270
   Arg Thr Thr Tyr Ala Asp Ala Val Lys Gly Arg Phe Ala Met Ser Arg
           275                 280                 285
   Asp Lys Ala Lys Ser Thr Val Phe Leu Gln Asn Ser Leu Lys Pro Glu
       290                 295                 300
   Asp Thr Ala Val Tyr Tyr Cys Gly Asp Val Pro Phe Ser Thr Leu Pro
   305 310 315 320
   Ala Met Cys Thr Asn Asp Gly Pro Trp Gly Gln Gly Thr Gln Val Thr
   325 330 335
   Val Ser Ser His His His His His His
   340 345

## Description

The present invention relates to a modified α-helical bundle cytokine, with reduced activity via a α-helical bundle cytokine receptor, wherein said α-helical bundle cytokine is specifically delivered to target cells. Preferably, said α-helical bundle cytokine is a mutant, more preferably it is a mutant interferon, with low affinity to the interferon receptor, wherein said mutant interferon is specifically delivered to target cells. The targeting is realized by fusion of the modified α-helical bundle cytokine to a targeting moiety, preferably an antibody. The invention relates further to the use of such targeted modified α-helical bundle cytokine to treat diseases. A preferred embodiment is the use of a targeted mutant interferon, to treat diseases, preferably viral diseases and tumors.

Cytokines are small proteins that play an important role in intercellular communication. Cytokines can be classified based on their structure, the largest group being the four-α-helix bundle family. This family can, based on the use of receptors, further be divided in the interferon (IFN) and interleukin (IL)-2, -3, -10 and -12 subfamilies. The a helical bundle cytokines are important as possible biopharmaceutical for treatment of human diseases; as a non-limiting example erythropoietin is used for treatment of anemia or red blood cell deficiency, somatotropin for treatment of growth hormone deficiency and interleukin-2 in the treatment of cancer.
Within the α helical bundle cytokines, type I IFNs belong to a cytokine family having important biological functions. In human, there are 17 different type I IFNs (13α, β, ε, κ, ω) which signal through an ubiquitously expressed cell surface receptor composed of two chains IFNAR1 and IFNAR2. The assembling of the IFN-receptor complex initiates the activation of several signal transduction pathways that, depending of the cell type, modify cellular differentiation and/or functions.
By acting on virtually every cell type, type I IFN is able to prevent productive viral infection. In addition, it exhibits marked antiangiogenic and proapoptotic effects. Type I IFNs are also deeply implicated in the regulation of several functions of the innate and adaptive immunity as well as on bone homeostasis. It acts particularly on the activation/differentiation of dendritic cells and osteoclasts. The type I IFN system is in fact critically important for the health of mammals.
Preclinical studies in mice have established a remarkable efficacy of type I IFN for the treatment of both viral or tumor diseases. Noteworthy, mice cured of an experimental tumor by IFN treatment have been found immunized against the initial tumor, suggesting that IFN acts not only to engage the processes of tumor rejection but also to break the immune tolerance against the tumor. Based on these studies, IFNα was approved in clinic for the treatment of both viral infection and cancer. More recently, IFN3 was shown to be effective in relapsing- remitting multiple sclerosis and was also approved for this pathology. Unfortunately, the clinical efficacy of IFN was often found disappointing and today other therapeutic strategies such as specific antiviral compounds, chemotherapies and monoclonal antibodies have, when possible, largely supplanted IFN broad application. Today, IFN is the first line therapeutic choice for only HBV and HCV chronic infections and for a limited number of tumors.
The efficacy of type I IFN in clinical practice is limited by ineffective dosing due to significant systemic toxicity and side effects, including flu-like syndrome, depression, hepatotoxicity, autoimmune disease, thyroid dysfunction and weight loss. It would thus be highly worthwhile to target IFN activity toward only the cellular population which should be treated with IFN (e.g. infected organ or tumor mass) or activated by IFN (e.g. subsets of immune cells).
In order to solve or limit the systemic toxicity of cytokines, specific targeting of cytokines by antibody-cytokine fusion proteins has been proposed (Ortiz-Sanchez et al., 2008). Rossi et al. (2009) specifically discloses CD20-targeted tetrameric IFNα, and its use in B-cell lymphoma therapy. However, the fusion maintains its biological activity, and is even more active than commercial pegylated IFN, which means that the unwanted side effects in human treatment would still be present, or would even be more severe. WO2009039409 discloses targeted IFN and its apoptotic and anti-tumor activities. The patent application discloses the fusion of an antibody as targeting moiety with wild type IFN, but also with mutated IFN. However, it is stated that the IFN fragment should retain its endogeneous activity at a level of at least 80%, or even at a higher level than wild type IFN. Also in this case, the fusion is retaining the unwanted side effects of the wild type.
WO2008/124086 discloses fusion proteins of a targeting element and a mutant IFN.
Surprisingly we found that a modified α-helical bundle cytokine, with a decreased affinity for the α-helical bundle cytokine receptor and a consequent decreased specific bioactivity can be fused to a targeting moiety, wherein the bioactivity is restored towards the targeted cells, but not towards cells that are not targeted by the construct. Such construct has the advantage over the art of having less side effects, especially a lower systemic toxicity, while retaining the bioactivity against the target cells.
The invention is defined by the appended claims.

A first aspect of the disclosure is a targeting construct, comprising modified α-helical bundle cytokine, characterized by a reduced affinity for the α-helical bundle cytokine receptor, and a targeting moiety. α-helical bundle cytokines are known to the person skilled in the art, and include, but are not limited Cardiotrophin-like cytokine NNT-1, Ciliary neurothrophic factor, Macrophage colony stimulating factor, Granulocyte-macrophage colony stimulating factor, Granulocyte colony stimulating factor, Cardiotrophin-1, Erythropoietin, FLT3 ligand, Somatotropin, Interferon a-1, 2, 4, 5, 6, 7, 8, 10, 13, 14, 16, 17, 21 ,, Interferon β, Interferon γ, Interferon κ, Interferon ε, Interferon τ-1, Interferon ω-1, Interleukin 2, 3, 4, 5, 6, 7, 9, 10, 11, 12 α chain, 13, 15, 19, 20, 21 , 22, 23, 24, 26, 27, 28A, 29, 31, Stem cell factor, Leptin, Leukemia inhibitor factor, Oncostatin M, Prolactin, and Thrombopoietin. For a review on α-helical bundle cytokines, see Conklin (2004). A modified α-helical bundle cytokine means that the α-helical bundle cytokine has been changed to alter the affinity to the receptor, with as final result that the modified α-helical bundle cytokine has a reduced affinity for the receptor and a consequent reduced biological activity, as compared to the endogenous wild type cytokine that binds normally to the receptor. Said modification is a mutation decreasing the affinity of the α-helical bundle cytokine. A reduced affinity and a consequent reduced biological activity as used here means that the modified α-helical bundle cytokine has a biological activity of less than 70% of the biological activity of the α-helical bundle cytokine, even more preferably less than 60% of the biological activity of the α-helical bundle cytokine, more preferably less than 50% of the biological activity of the α-helical bundle cytokine, more preferably less than 40% of the biological activity of the α-helical bundle cytokine, more preferably less than 30% of the biological activity of the α-helical bundle cytokine, more preferably less than 20% of the biological activity of the α-helical bundle cytokine, most preferably less than 10% of the biological activity of the α-helical bundle cytokine as compared to the α-helical bundle cytokine that normally binds to the receptor. Preferably, the modified α-helical bundle cytokine is a mutant of the wild type α-helical bundle cytokine and the activity is compared with the wild type α-helical bundle cytokine. The affinity and/or the activity can be measured by any method known to the person skilled in the art. Preferably, the activity is measured by measuring and quantifying STAT phosphorylation.
The targeting construct of the invention is a mutant human IFNα2 characterized by reduced affinity for the IFN receptor, and a targeting moiety. A mutant human IFNα2 as used here is a mutant form that has a lower affinity for the receptor and as a consequence a lower antiproliferative activity and/or a lower antiviral activity. Indeed, as shown by Piehler et al. (2000), the relative affinity correlates directly with the relative antiproliferative activity and with the relative antiviral activity. The affinity of the mutant human IFNα2 to the receptor, in comparison to the affinity of the wild type human IFNα2 to the receptor can be measured by reflectometric interference spectroscopy under flow through conditions, as described by Brecht et al. (1993). The mutant may be a point mutant, a deletion or an insertion mutant, or a combination thereof. Preferably, said mutant human IFNα2 is obtained by active mutagenesis, such as, but not limited to site directed mutagenesis by polymerase chain reaction amplification. Said mutant human IFNα2 has a biological activity of less than 70% of the biological activity of the wild type IFNα2, even more preferably less than 60% of the biological activity of the wild type IFNα2, more preferably less than 50% of the biological activity of the wild IFNα2, more preferably less than 40% of the biological activity of the wild IFNα2, more preferably less than 30% of the biological activity of the wild IFNα2, more preferably less than 20% of the biological activity of the wild IFNα2, most preferably less than 10% of the biological activity of the wild type of which it is deduced (i.e. the wild type human IFNα2 of which the coding sequence has been mutated to obtain the mutant human IFNα2). Mutant forms of IFNα2 are known to the person skilled in the art. As a non-limiting example, IFNα2 mutants have been listed in Piehler et al. (2000). Said IFNα2 mutant is mutated in one or more amino acids of the region 144-154, preferably at position 148, 149 and/or 153, even more preferably, said mutant IFNα2 is selected from the group consisting of IFNα2 L153A, IFNα2 R149A and IFNα2 M148A. Most preferably said mutant is selected from the group consisting of IFNα2 L153A and IFNα2 R149A.
Preferably, said receptor is IFNAR2.
Said targeting moiety is directed to a marker selected from the group consisting of Her2, DC-STAMP, PDL2, and CD20.

A targeting moiety, as used here, is a variable domain of camelid heavy chain antibodies (VHH). Preferably, said targeting moiety consists of a single polypeptide chain and is not post-translationally modified. Even more preferably, said targeting moiety is a nanobody.
The targeting construct can be any targeting construct known to the person skilled in the art. As a non-limiting example, the targeting moiety may be chemically linked to the mutant interferon, or it may be a recombinant fusion protein. Preferably, said targeting construct is a recombinant fusion protein. The targeting moiety may be fused directly to the mutant IFN, or it may be fused with the help of a linker fragment. The targeting moiety may be fused at the aminoterminal or at the carboxyterminal end of the mutated IFN; preferably said targeting moiety is fused at the aminoterminal extremity of the mutated IFN molecule.
Another aspect of the invention is a targeting construct according to the invention for use as a medicament.
Another aspect of the invention is a targeting construct according to the invention for use in treatment of cancer.

Still another aspect of the invention is a targeting construct according to the invention for use in treatment of a viral disease. As a non-limiting example said viral disease may be HIV infection, HBV infection or HCV infection.
Still another aspect of the invention is a targeting construct according to the invention for use in treatment of diseases involving bone degradation, such as, but not limited to osteoporosis.
Disclosed is also a pharmaceutical composition, comprising a targeting construct according to the invention and a suitable excipient. It is clear for the person skilled in the art that such a pharmaceutical composition can be used alone, or in a combination treatment, such as, but not limited to a combination with chemotherapy.

### BRIEF DESCRIPTION OF THE FIGURES

Figure 1: Representation of the structural elements of the nanobody-IFN fusion protein.
Figure 2: Firefly luciferase activity induced by the indicated IFN preparation on HL116 cells (panels A and B) or HL116 cells expressing the murine leptin receptor (mLR) (panels C and D). Panels A and C on the one hand and panels B and D on the other hand were generated in two separate experiments. Consequently only vertical comparison (panel A versus panel C or panel B versus panel D) is possible.
Figure3: Renilla (light grey) and Firefly (dark grey) luciferase activity induced by the nanobody-IFNα2R149A or by the IFNα2 (7pM) in a 1:1 coculture of cells expressing the leptin receptor and an IFN-inducible firefly luciferase or in cells expressing an IFN-inducible renilla luciferase but devoid of leptin receptor. Luciferase activities are expressed as a percentage of the luciferase activities induced by 3 nM IFN α2.
Figure 4: Activity of the purified constructs targeting the mLR: A. Quantification of their specific activities on cells expressing the target (HL116-mLR) or on cells lacking the target (HL116). B. Calculation of the targeting efficiencies of the different constructs.
Figure 5: Activity of the construct 4-11-IFNA2-R149A in presence and absence of the unconjugated leptin receptor binding nanobody. HL116 cells expressing the mLR were incubated for 6 hours with either the IFN-α2 (IFNA2) or the IFNA2-R149A fused to the nanobody 4-11 (Nanobody-IFNA2-R149A) at their respective EC50 concentration in the presence or absence (control) of a 100-fold molar excess of free 4-11 nanobody.
Figure 6: Targeting the mutant IFN using the leptin binding nanobody 4-10.
Figure 7: Firefly luciferase activity induced in HL116 cells expressing the mLR by the nanobody-IFNα2R149A in the presence of anti IFNAR1 monoclonal antibody 64G12 (Benoit et al. J. Immunol. 150, 707-716. 1993) or anti IFNAR2 monoclonal antibody MMHAR2 (PBL Interferon Source)

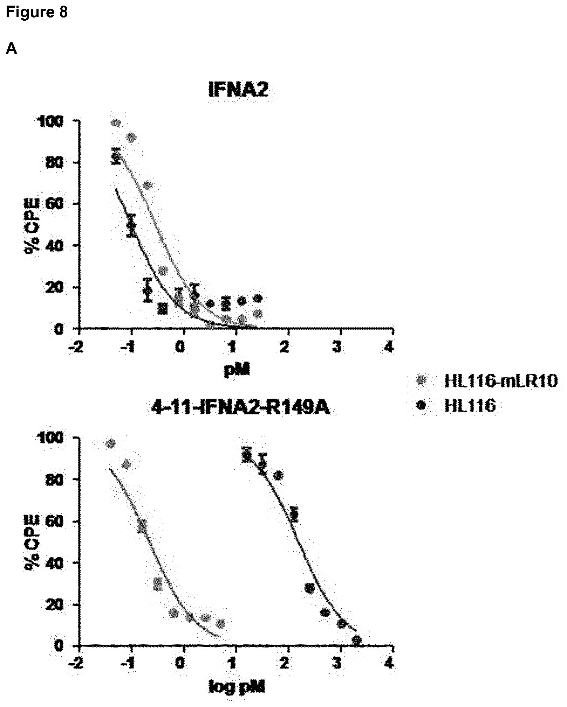

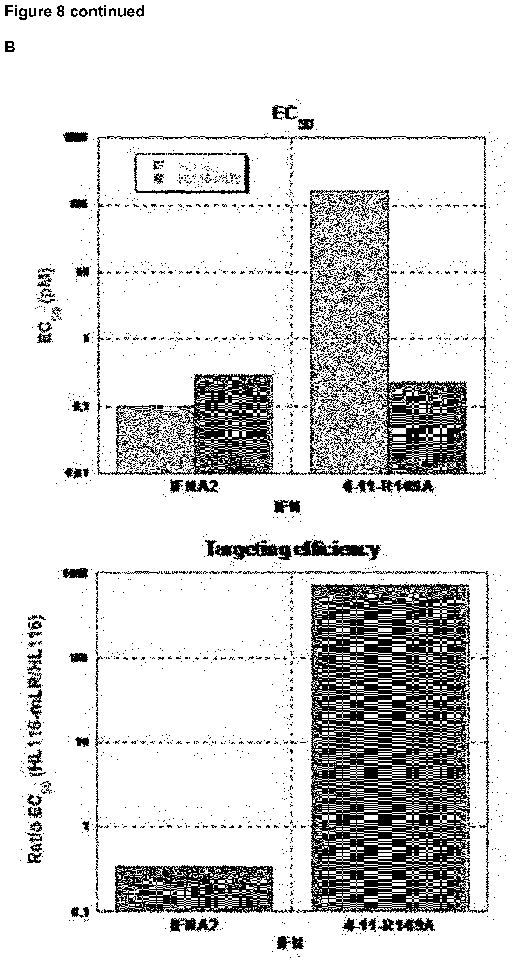

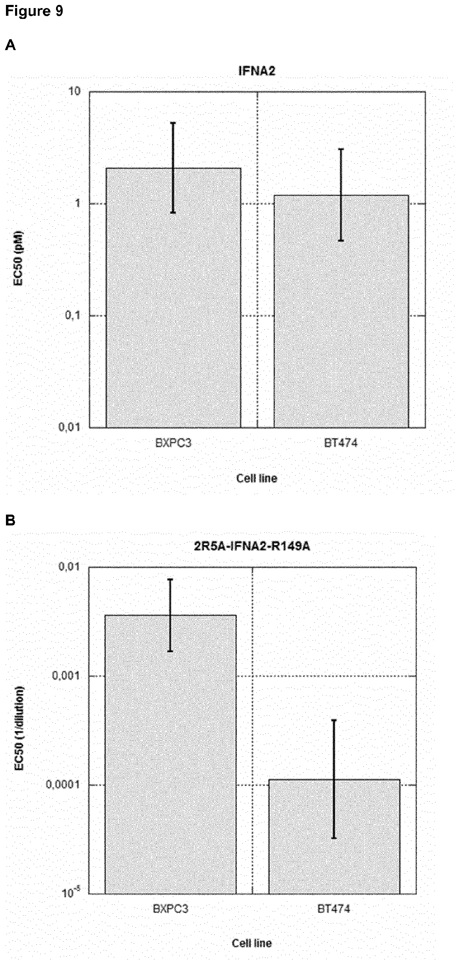

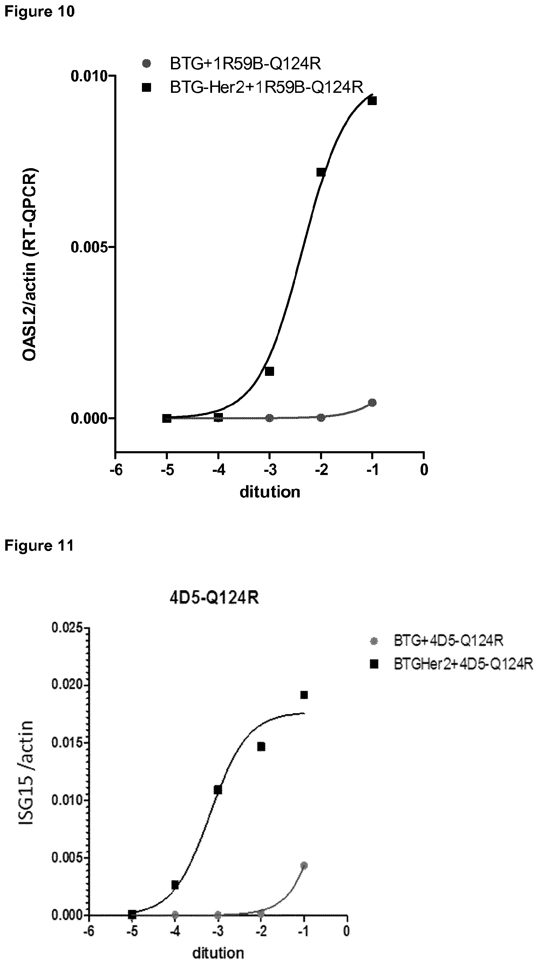

Figure 8: Specificity of the targeting to of 4-11-IFNA2-R149A to cells expressing the mLR. A: Cytopathic effect of the EMCV on HL116 cells (dark gray symbols) or on HL116-mLR (light grey symbols) of parental IFNA2 (upper left panel) or of the 4-11-IFNA2-R149A (lower left panel). B: Upper panel: calculated EC50 for antiviral activity; lower panel: calculated targeting efficiencies Figure 9: Specific activities (EC50) of IFNα2 (panel A) and the nanobody-IFNα2R149A (2R5A; panel B) on BXPC3 and BT474 cell lines which express different number of Her2 molecule at their surface (10.9 x 10<3> and 478 x 10<3>, respectively). The ordinate scale of panel A cannot be compared to the ordinate scale of panel B. Figure 10: Targeting of the 1 R59B-IFNA2-Q124R to human Her2 expressing mouse cells. Quantification of the OASL2 mRNA expression in BTG9A cells with and without Her2 expression.
Figure 11: Targeting of mutant IFNA2 to human Her2 expressing mouse cells, using a single chain antibody. Quantification of the ISG15 mRNA expression in BTG9A cells with and without Her2 expression.
Figure 12: Control of the activation of Her2 phosphorylation: Lane 1 3 to 76: no phosphorylated Her2 in extract of BTG9A cells expressing human Her2 treated with different concentration (200 pM for lane 3 to 5, 2 nM for lane 6) and time (lane 3: 5 min, lane 4 and 6: 10 min, lane 5: 30 min.) with the construct 1 R59B-IFNA2-Q124R.
   Lane 7 and 8: control for the detection of phosphorylated Her2 in the human BT474 cell line.
   Lane 1 extract of BTG9A cells. Lane2: extract of BTG9A cells expressing human Her2.
Figure 13: Targeting the anti PD-L2 122-1 FNA2-Q124R to mouse primary cells endogenously expressing PD-L2. The activation is measured as STAT phosphorylation. The light gray area represents the PD-L2 negative cell population; the dark gray area represents the PD-L2 positive population.
Figure 14: In vivo targeting of 122-1 FNA2-Q124R to PD-L2 expressing cells. Mice were injected intraperitoneally (IP) or intravenous (IV) with either PBS, a control construct (nanobody against GFP fused to mutant IFNA2-Q124R, indicated as control) or a targeted mutant IFN (targeted to PD-L2, NM22-IFN2-Q124R, indicated as 122-Q124R. The light gray area represents the PD-L2 negative cell population; the dark gray area represents the PD-L2 positive population.

Figure 15: Dose response curve after IV injection of 122-IFN-Q124R in mice. The light gray area represents the PD-L2 negative cell population; the dark gray area represents the PD-L2 positive population.
Figure 16: Leptin-dependent growth induced by targeted mutant leptin: the loss in activity of a mutant leptin can be rescued in Ba/F3 cells expressing the human TNFR1 . Upper panels: experiment using the H6-leptin construct; lower panel, experiment using the mleptin construct. H6 indicated the his tag (6 xhis).
Figure 17: construction of the targeted leptin constructs

### EXAMPLES

### Materials & Methods to the examples

### Nanobodies and ScFv

The nanobody 4-11 directed against the murine leptin receptor was described in Zabeau et al. (2012), and in the patent WO 2006/053883. Its coding sequence is cloned into the mammalian expression vector pMET7 (Takebe et al., 1988) in fusion with the Slgk leader peptide, the HA tag and albumin. Plasmid name: pMET7 SlgK-HA-4.11-Albumin.
The nanobody 4-10 is also described in Zabeau et al. (2012).
The anti Her2 nanobodies 1R59B and 2R5A are described in Vaneycken et al. (2011). They were fused to the human IFNA2-Q124R and to the human IFNA2-R149A in the pMET7 vector. Fusion protein was produced by transfection of 293T cells.
The anti PD-L2 nanobody 122 was from Johan Grooten (VI B). It was fused to the human IFNA2-Q124R in the pMET7 vector. The fusion protein was produced by transfection of 293T cells and purified using the HisPur Ni-NTA purification kit (Pierce, Thermo Scientific).
The anti TNF nanobody was obtained from Claude Libert (VIB).

The anti Her2 ScFv was obtained from Andrea Pluckthun (Wörn et al., 1998). It was fused to the human IFNA2-Q124R in the pMET7 vector. The fusion protein was produced by transfection of 293T cells.

Control nanobody against GFP was obtained from Katrien Van Impe (University Ghent)

### Interferons

The IFNα2 and the mutants L153A and R149A which show an IFNAR2 affinity reduced by a factor 10 and 100, respectively, have been described in Roisman et al., (2001). IFN coding sequences are cloned in the pT3T7 vector (Stratagene) in fusion with the ybbR tag. Plasmid names: pT7T3ybbR-IFNα2, pT7T3ybbR-IFNα2-L153A, pT7T3ybbR-IFNα2-R149A.
The human IFNA2 Q124R has a high affinity for the murine IFNAR1 chain and a low affinity for the murine IFNAR2 chain. (Weber et al., 1987)

### Nanobody-IFN fusion construction

The coding sequence of the IFNα2, wild type, L153A and R149A were synthesized by PCR from the corresponding pT3T7ybbR IFNα2 plasmids using the Expand High Fidelity PCR system from Roche Diagnostics and the following primers: Forward:
5'GGGGGGTCCGGACCATCACCATCACCATCACCATCACCATCACCCTGCTTCTCCCGCCTCCCCAGCATCACCT GCCAGCCCAGCAAGTGATAGCCTGGAATTTATTGC3', Reverse: 5' CGTCTAGATCATTCCTTACTTCTTAAA C3'. This PCR introduces a His tag and a series of 5 Proline - Alanine - Serine (PAS) repeats at the amino terminal extremity of the IFNs. The PCR products were digested with BspEI and Xbal and cloned into BspEI-Xbal digested pMET7 SlgK-HA-4.11-Albumin vector to obtain pMET7 SlgK-HA-4.11-His-PAS-ybbr-IFNA2, pMET7 SlgK-HA-4.11-His-PAS-ybbr-IFNA2-L153A and pMET7 SlgK-HA-4.11-His-PAS-ybbr-IFNA2-R149A.
In a similar way the human mutant Q124R was fused to the 1 R59B nanobody and to the anti-PD-L2 nanobody.

### Production of the nanobody-IFN fusion protein

HEK293T cells were grown in DMEM supplemented with 10% FCS. They were transfected with pMET7 SlgK-HA-4.11-His-PAS-ybbr-IFNA2, pMET7 SlgK-HA-4.11-His-PAS-ybbr-IFNA2-L153A pMET7 SlgK-HA-4.11-His-PAS-ybbr-IFNA2-R149A, pMET7 SlgK-HA-2R5A-His-PAS-ybbr-IFNA2-R149A, pMET7 SlgK-HA-1R59B-His-PAS-ybbr-IFNA2-Q124R, pMET7 SlgK-HA-4D5-His-PAS-ybbr-IFNA2-Q124R or pMET7 SlgK-HA-122-His-PAS-ybbr-IFNA2-Q124R using lipofectamin (Invitrogen). 48 hours after the transfection culture mediums were harvested and stored at -20°C.
Alternatively, sequences encoding the different nanobody-IFN fusions where subcloned into the baculovirus transfer plasmid pBAC-3 (Novagen). Proteins were produced by insect cells using the BacVector kit (Novagen) and purified to homogeneity using the HisPur Ni-NTA purification kit (Pierce, Thermo Scientific) and gel filtration. Protein concentrations were measured by absorbance at 280 nm.

### IFN reporter cell lines

The HL116 clone (Uzé et al. 1994) is derived from the human HT1080 cell line. It contains the firefly luciferase gene controlled by the IFN-inducible 6-16 promoter. The HL116 cells were co- transfected with an expression vector encoding the short isoform of the murine leptin receptor (pMET7 mLRsh-FLAG, Eyckerman et al., 1999) and pSV2neo (Southern and Berg 1982). Stable transfected clones were isolated in G418 containing medium. The clone 10 was selected after analysis of the surface expression level of the murine leptin receptor by FACS, using the the biotinylated anti-mouse leptin receptor antibody BAF497 from R&D and streptavidin-APC (BD bioscience)
HT1080 cells were cotransfected with p6-16-RL, a plasmid encoding the Renilla luciferase (from pRL-null, Promega) controlled by the IFN-inducible 6-16 promoter (from p1.8gpt-5, Pellegrini et al. 1989), pBB3 (Bourachot et al. 1982) and salmon sperm DNA (Sigma). Stable transfected clones were isolated in HAT containing medium. The clone 4 was selected for a high level of renilla luciferase activity induction upon IFN induction.
The human pancreatic carcinoma BXPC3 (Tan et al., 1986; ATCC: CRL 1687) and breast cancer BT474 (Lasfargues et al., 1979; ATCC: HTB-20) cell lines were obtained from ATCC. The mouse BTG9A cells were described in Uzé et al. (1990).

### Measurement of the luciferase activities

IFN specific activities were measured by quantifying the luciferase activity induced in HL116 cells and on the HL116 clone 10 expressing the mLR. The EC50 were calculated using nonlinear data regression with GraphPad Prism software.
Luciferase activities were determined on a Berthold centra LB960 luminometer using either the Firefly Luciferase Assay System or the Dual-Luciferase Reporter Assay System from Promega after 6hr IFN stimulation.

### Quantitative RT-PCR

The expression of the interferon inducible gene 6-16 was quantified by RT-PCR relatively to GAPDH or β-actin. Cells were treated with targeted or control IFN for 4 hr. Total RNA was purified with RNeasy columns (Qiagen). Reverse transcriptions were primed with random primers and performed using Moloney murine leukemia virus reverse transcriptase (Invitrogen). Quantitative real-time PCR (qRT-PCR) was performed using a Light Cycler as described (Coccia et al. 2004).
For Her2, the transfection culture medium was assayed on murine BTG9A and BTG9A cells expressing human Her2 for expression of the OASL2 gene relatively to the expression of the β actin gene by quantitative RT-PCR using a Light Cycler (Roche) and the following primers: OASL2 forward: CAC-GAC-TGT-AGG-CCC-CAG-CGA; OASL2 reverse: AGC-AGC-TGT-CTC-TCC-CCT-CCG; 3actin forward: AGA-GGG-AAA-TCG-TGC-GTG-AC; 3actin reverse: CAA-TAG-TGA-TGA-CCT-GGC-CGT. In a similar way the ISG expression in Her2 targeted cells was measured using the same 3actin primers and the following primer ISG15 primers: ISG15 forward: GAG-CTA-GAG-CCT-GCA-GCA-AT; ISG15 reverse: TTC-TGG-GCA-ATC- TGC-TTC-TT.

### Antiviral Assay

The antiviral assay was performed using the EMC virus and scoring the virus replication dependent cytopathic effect as described in Stewart (1979)

### Measurement of Her2 phosphorylation

BTG9A cells expressing human Her2 were treated with 200 pM to 2 nM of 1 R59B-IFNA2- Q124R for 10 to 30 min. Cells were lysed in RIPA, and analysed by western blot on an Odyssey Fc (Licor Bioscience) after 7% SDS-PAGE (40 µg lane). Phopho-Her2 was detected with the anti Her2 Y-P 1248 (Upstate #06-229) and the Goat anti rabbit secondary antibody IRDye 680 (Licor Bioscience #926-32221).

### Measurement of STAT1 phosphorylation

STAT1 phosporylated on Y701 were detected by FACS using the STAT1 -PY701 (PE) (Beckton Dickinston #612564) and the manufacturer instruction for the PhosFlow technology.

### Targeted leptin constructs

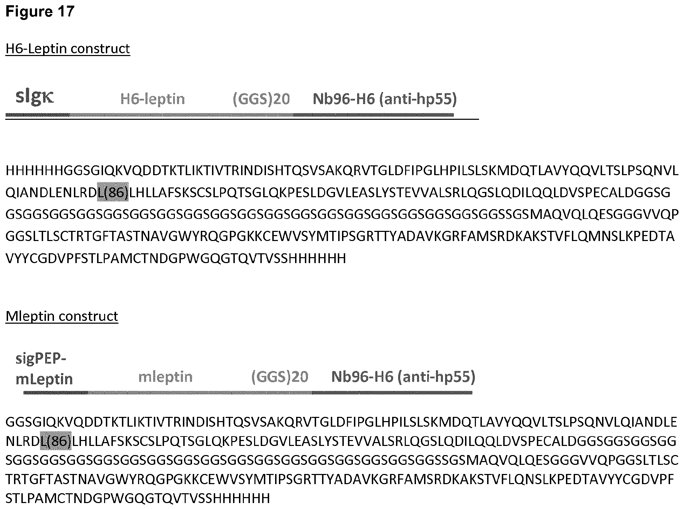

The sequence of the targeted leptin constructs is given in Figure 17. The L86 which is indicated is the amino acid that is mutated either to S or N.

### Example 1: The nanobody-interferon fusion proteins

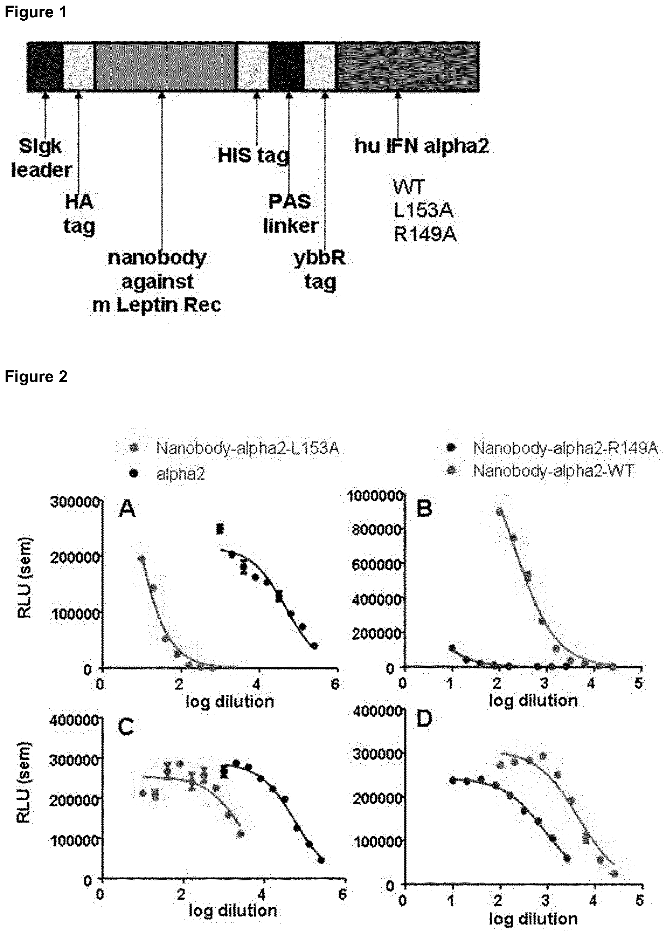

Figure 1 shows a schematic representation of the nanobody-IFN fusion proteins constructed with either IFNα2 wild type or the L153A and R149A mutants.

### Example 2: IFN activity of the nanobody-IFN fusion proteins is targeted toward murine leptin receptor expressing cells (for illustration only)

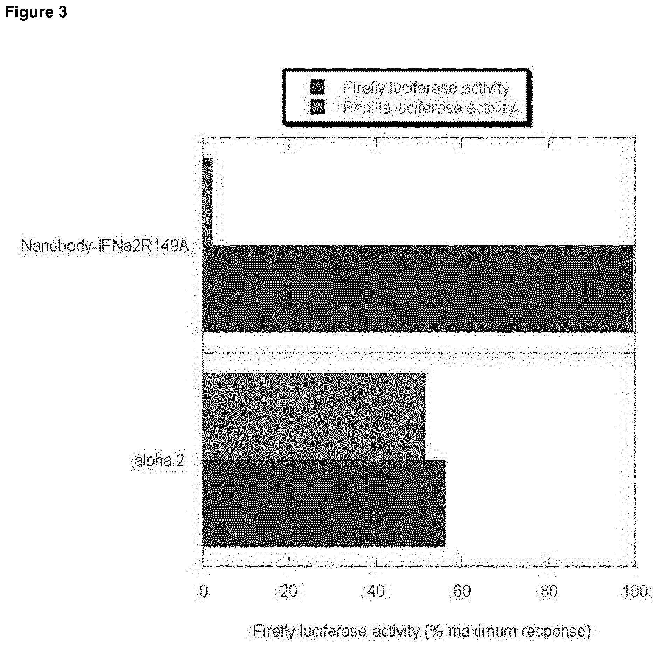

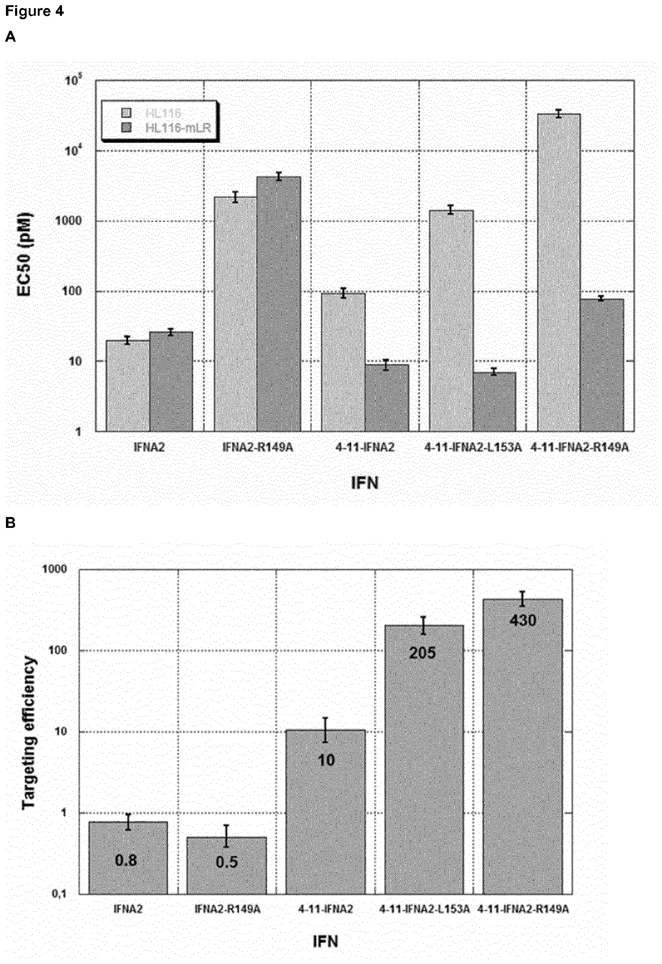

The three nanobody fusion proteins with IFNα2 WT, IFNα2 L153A or R149A were assayed on both HL116 and HL116-mLR-clone 10 cells which express the murine leptin receptor. The IFNα2 alone was also assayed in this assay system in order to check that the two cell clones do not differ in their IFN responsiveness. Indeed both HL116 and HL116-mLR-clone 10 cells are equally sensitive to this IFN (Fig 2A and 2C, black symbols). The IFN activity of the three nanobody-IFN fusion proteins is however dramatically increased in cells expressing the leptin receptor compared to parental HL116 cells (compare Fig. 2A with Fig. 2C and Fig. 2B with Fig. 2D).
We estimated that cells expressing the leptin receptor are 10, 100 and 1000-fold more sensitive than parental HL116 cells to the nanobody-IFN WT, L153A and R149A, respectively. Since the affinities for IFNAR2 of the IFN mutant L153A and R149A are 0.1 and 0.01 relatively to the WT, there is a correlation between the loss of activity caused by mutations in the IFNAR2 binding site and the targeting efficiency by the nanobody.
In order to determine whether the IFN activity of the nanobody-IFN fusion proteins is delivered only on cells expressing the nanobody target or also on neighboring cells, the nanobody-IFNα2R149A was assayed on a coculture of HL116-mLR-clone10 and HT1080-6-16 renilla luciferase clone4. Both cell types will express luciferase activity in response to IFN stimulation but cells expressing the target of the nanobody will display a firefly luciferase activity whereas cells devoid of leptin receptor will display a renilla luciferase activity. The dilution of the nanobody-IFNα2R149A protein was chosen at 1/30, a dilution which induces a maximal response in cells carrying the leptin receptor and a minimal response on cells devoid of the nanobody target (see Fig.2B and D, black curves). Fig. 3 shows clearly that the renilla luciferase activity is not induced upon stimulation of the co-culture with the nanobody-IFNα2R149A, indicating that the targeted IFN activity is delivered only on cells expressing the antigen recognized by the nanobody.
The efficacy of the targeting is further illustrated by comparing the activity of wild type and two types of mutant IFN (L153A and R149A) when added to HL116 expressing or not expressing the murine leptin receptor that is used for the targeting. The results clearly show that the activity of the mutants is higher when the construct is targeted, and that the effect of targeting for the mutant is bigger than for wild type. (Figure 4A and B)

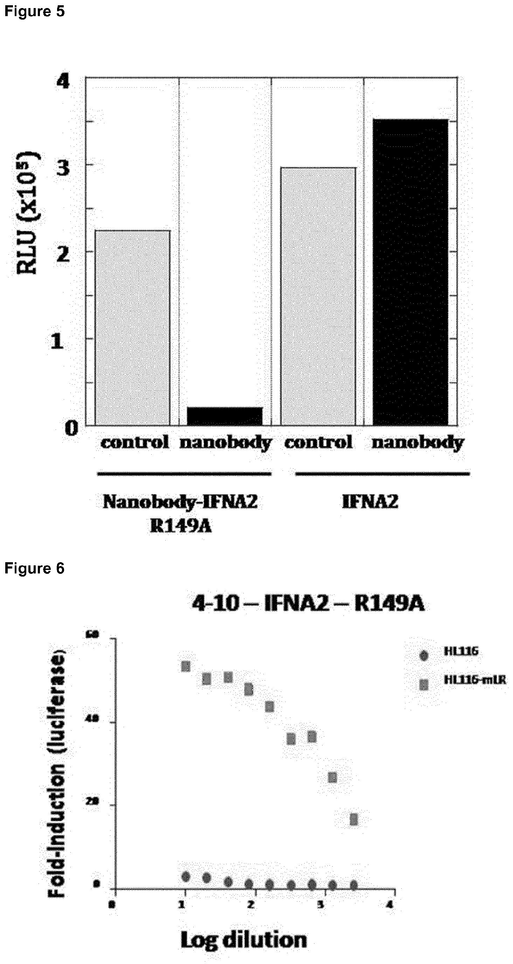

In order to prove that the targeting was nanobody specific, HL116 cells expressing the mLR were incubated for 6 hours with either the IFN-a2 (indicated as IFNA2) or the IFNA2-R149A fused to the nanobody 4-11 (Nanobody-IFNA2-R149A) at their respective EC50 concentration in the presence or absence (control) of a 100-fold molar excess of free 4-11 nanobody. Cells were lysed and the IFN-induced luciferase activities were measured. As shown in Figure 5, the non-targeted IFN is not inhibited by the free nanobody, while the targeted construct is strongly inhibited, showing the specific effect of the targeting.
The targeting to the leptin receptor is independent of the epitope on the receptor: using the anti-leptin receptor nanobody 4-10 (Zabeau et al., 2012) which recognizes a different domain on the receptor than the nanobody 4-11, a similar activation can be obtained using a targeted mutant IFN (Figure 6).

### Example 3: The IFN activity of the nanobody-IFN fusion proteins on cells expressing the leptin receptor is mediated by both IFN receptor chains (for illustration only)

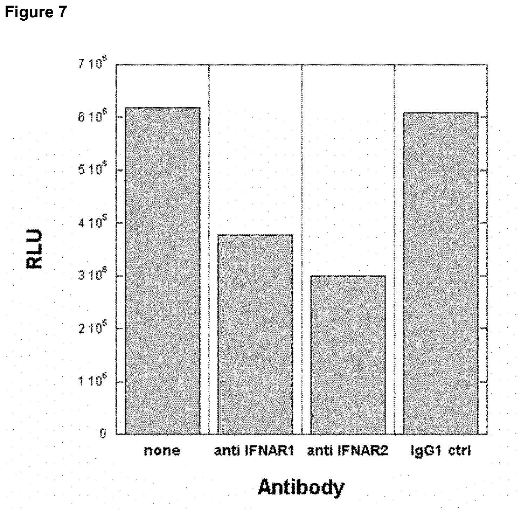

In order to determine whether the IFN activity of the nanobody-IFN fusion proteins needs the activation of the IFN receptor, HL116 cells expressing the murine leptin receptor were pretreated with neutralizing antibodies against IFNAR1 or IFNAR2, and then stimulated with the nanobody-IFNA2-R149A fusion protein. The activity of the IFN-induced luciferase was measured. The figure 7 shows that both anti IFNAR1 and anti IFNAR2 neutralizing antibodies inhibit the IFN activity of the nanobody-IFNA2-R149A.

### Example 4: Target-specific induction of antiviral activity by 4-11-IFNA2-R149A in cells expressing the murine leptin receptor (for illustration only)

Antiviral activity is an integrated part of the IFN response, implying the expression of several genes. Therefore, the antiviral activity on mLR expressing cell was controlled, after targeting the mutant R149A IFN using the anti-leptin receptor antibody 4-11. The results are summarized in Figure 8. The activity was measured as the cytopathic effect on HL116 cells, with or without leptin receptor expression. The specific antiviral activity of the 4-11-IFNA2-R149A nanobody-IFN fusion protein is 716-fold higher when assayed on leptin receptor expressing cells compared to HL116 cells.

### Example 5: Targeting of IFN activity on Her2 expressing cells

In order to demonstrate that the concept is not restricted to cytokine receptor targeting, we generated similar fusion protein using the nanobody 2R5A against Her2 (Vaneycken et al., 2011) and the mutant IFN alpha2 R149A (2R5A-IFNA2-R149A). This molecule was assayed on BXPC3 (Pancreatic cancer, from ATCC) and BT474 (Breast cancer, from ATCC) cell lines and compared with the activity of IFN-a2 (IFNA2) for the induction of the 6-16 IFN-inducible gene as determined relatively to GAPDH by quantitative RT-PCR. The BXPC3 and BT474 cells lines differ by their number of Her2 molecules expressed at their surface (10.9 x 10³ and 478 x 10³, respectively as reported by Gaborit et al. (2011)). Figure 9 shows the EC₅₀ determination of IFNA2 activity (Panel A) and 2R5A-IFNA2-R149A activity (Panel B) for the induction of the IFN-inducible gene 6-16 on BXPC3 and BT474 cell lines. Panel A shows that BXPC3 and BT474 cell lines exhibit the same sensitivity to IFN-α2. Panel B shows that the 2R5A-IFNA2-R149A nanobody-IFN fusion protein is much more potent on the BT474 cell line which expresses 40-fold more Her2 molecule than BXPC3.
In conclusion, the concept which consists to target type I IFN activity on cells expressing a specific cell surface antigen, as shown on human cells expressing the mouse leptin receptor, can be extended to untransfected human cells expressing another cell surface molecule from a different structural family, at a level naturally found in several types of breast carcinoma.

### Example 6: Targeting of mutant IFNA2-Q149R to mouse cells expressing human Her2

Mutant human IFNA2 Q149R was targeted to murine cells, expressing the human Her2, using the nanobody 1 R59B in the 1 R59B-IFNA2-Q124R. The IFNA2 Q124R has a high affinity for the murine IFNAR1 chain and a low activity for the murine IFNAR2 chain (Weber et al., 1987). The induction by IFN was measured as expression of the OASL2 messenger RNA, by RT- QPCR. The results are shown in Figure 10. There is clearly a targeting-specific induction in the Her2 expressing cells, whereas there is no significant expression detected in untransfected BTG9A cells.
Similar results were obtained when the Her2 specific ScFv against Her2 was used to target the mutant IFN Q124R. In this case, the IFN induction was measured using the ISG15 messenger RNA expression. The results are shown in Figure 11. Again, a specific induction of ISG15 is seen in the cells expressing Her2, while there is little effect of the mutant IFN on the cells that do not express Her2.

### Example 7: The construct 1 R59B-IFNA2-Q124R does not activate the phosphorylation of Her2

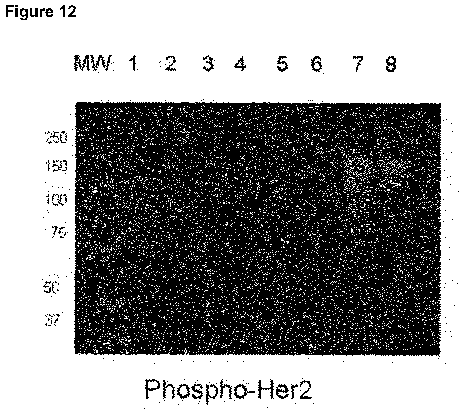

To check whether targeting of Her2 is resulting in a Her2 activation, Her2 phosphorylation was controlled in targeted cells. The results are shown in Figure 12, clearly demonstrating that no phosphorylated Her2 could be detected in 1 R59B-IFNA2-Q124R targeted cells, irrespective of the concentration or time of treatment.

### Example 8: The anti PD-L2 Nb122-IFNA2-Q124R construct activity is targeted on mouse primary cells expressing PD-L2

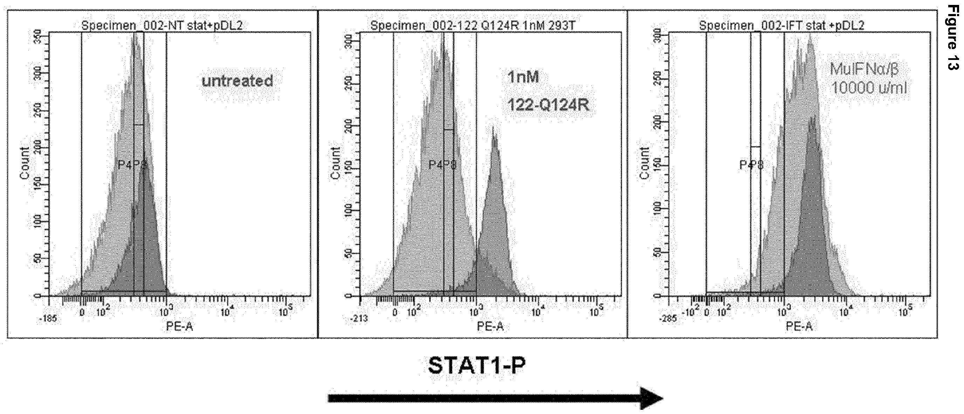

Cells from a mouse peritoneal cavity were isolated and treated in vitro with Nb122-IFNA2- Q124R or natural mlFNa/β for 30 min. Cells were, fixed, permeabilized, labelled with antibodies against PD-L2 (APC) (BD #560086) and STAT1 -PY701 (PE) (BD #612564) and analysed by FACS.
The PD-L2 positive cell population represents 20% of the total cell population present in the mouse peritoneal cavity.
The results are shown in Figure 13. It is clear from this figure that in untreated cells, or in non-targeted, murine IFN treated cells the peaks of STAT1-P for PD-L2 expressing and non-expressing cells coincide. Moreover, a clear induction in STAT1-P can be seen by murine IFN treatment. Treatment with the targeted mutant IFN however results in a specific shift in the STAT1-P only for the PD-L2 expressing cells.
A same result is obtained if the IFN response of splenocytes is analysed in a similar experiment. The PD-L2 positive cell population represents 1 % of the total cell population present in mouse spleen, indicating that also a minor cell population can be targeted in an efficient way.

### Example 9: In vivo injection of 122-IFNA2-Q124R construct induces an IFN response only in PD-L2 - expressing cells

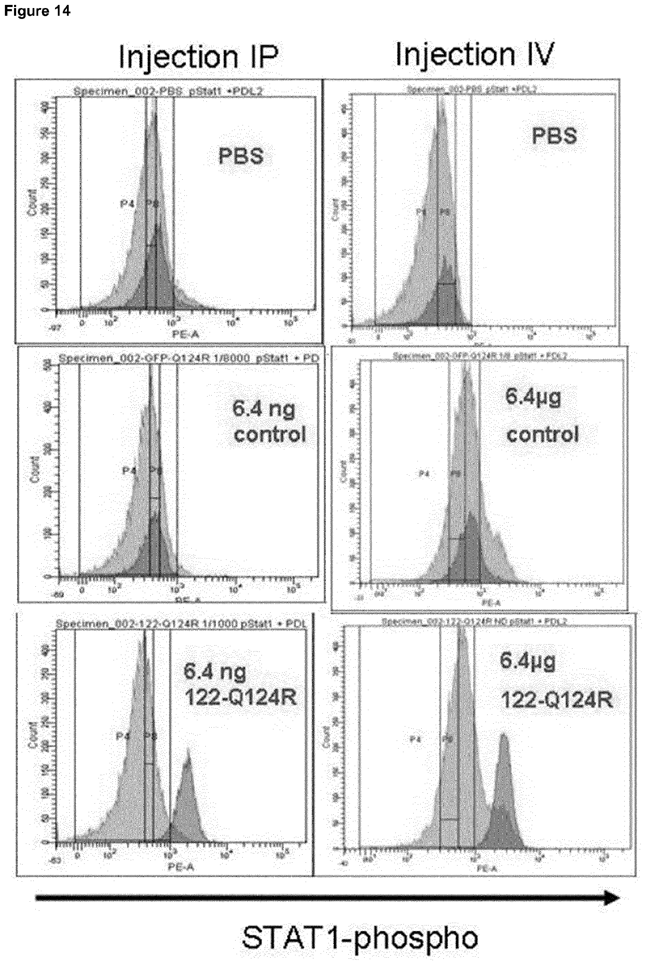

Mice were injected (IP or IV) with either PBS, Nbl22-IFNA2-Q124R or a control Nb (against GFP) fused to IFNA2-Q124R. 30 min post injection, mice were killed, cells from the peritoneal cavity were recovered by washing the peritoneal cavity with PBS, fixed (PhosLow Fix buffer I BD # 557870), permeabilized (PhosFlow Perm buffer III, BD #558050), labelled with Abs against PD-L2 (APC) (BD #560086) and STAT1 -PY701 (PE) (BD #612564) and analysed by FACS. The results are shown in Figure 14. STAT1 -P coincides in PD-L2 positive and negative cells treated with either PBS or control nanobody. However, a clear induction in STAT1 -P (only in the PDL2 positive cell population) can be seen when the mice are injected with the targeted mutant IFN.
As a control, STAT1 -P was checked in mice, iv injected with different doses of natural mouse IFN (10 000, 100 000 or 1 000 000 units), and no difference in STAT1-P could be detected between the PD-L2 positive and PD-L2 negative cells.

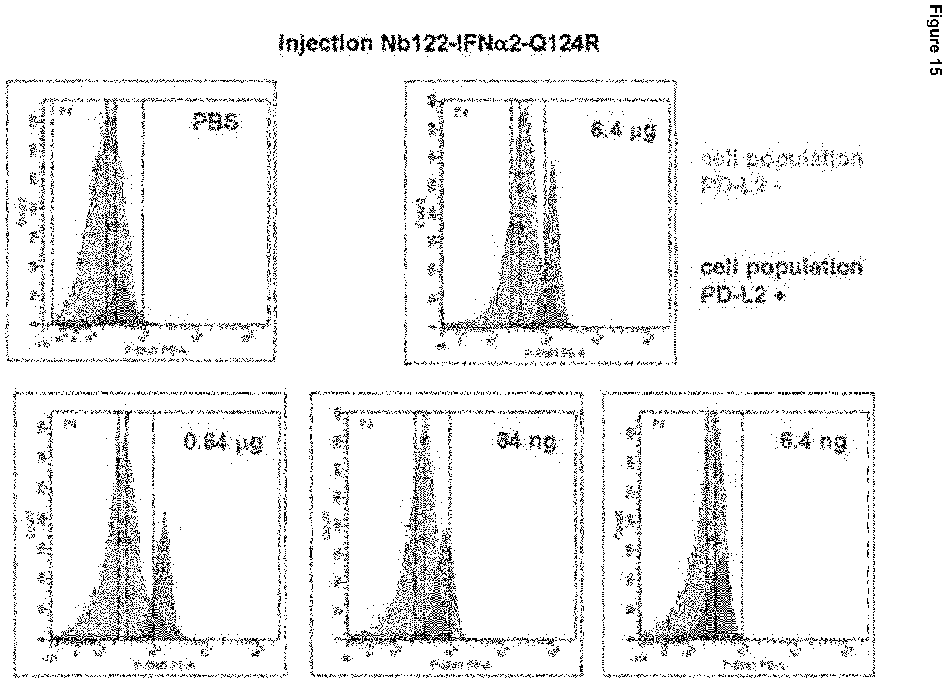

Figure 15 shows a similar dose response curve after iv injection of the Nbl22-IFNA2-Q124R construct. A shift in STAT1 -P in the PD-L2 expressing cells can be noticed even at the lowest dose of 64ng.

### Example 10: Targeting of mutant leptin to the leptin receptor, using a truncated TNFα receptor (for illustration only)

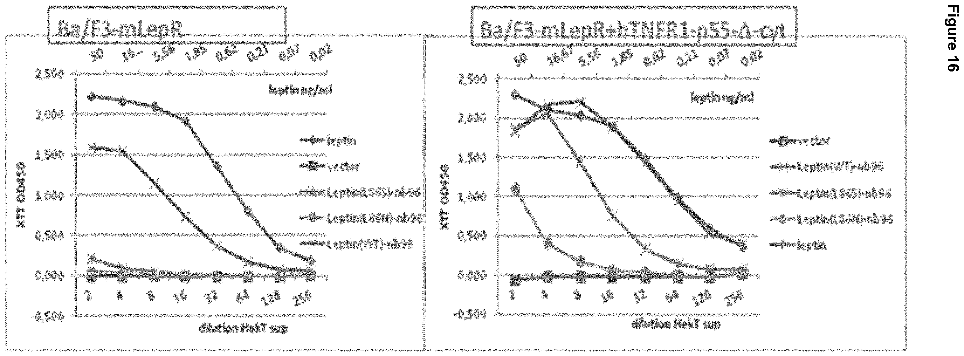

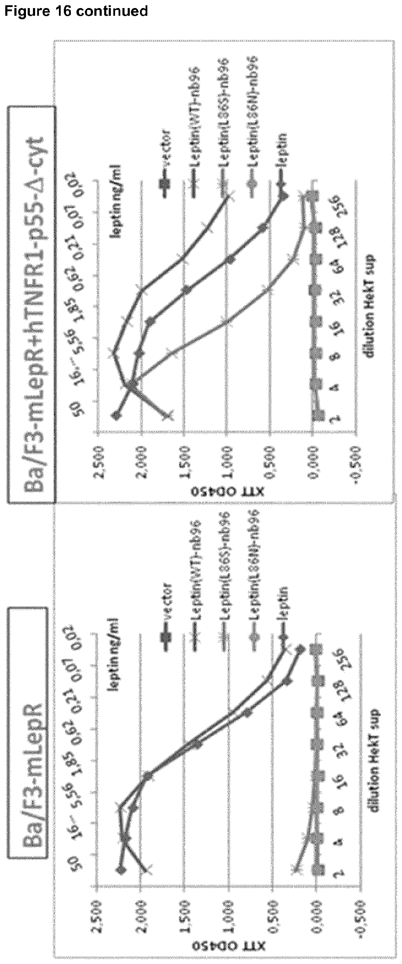

Ba/F3 cells are growth-dependent on IL-3. After transfection with the mLR, Ba/F3 cells also proliferate with leptin. Leptin mutants with reduced affinity for their receptor are less potent in inducing and sustaining proliferation of Ba/F3-mLR cells. Leptin mutant L86S has a moderate and mutant L86N has a strong reduction in affinity and hence a moderate and strong reduced capacity to induce proliferation respectively.
Additional transfection of Ba/F3-mLR cells with the human TNFα Receptor 1 (hTNFR1) lacking its intracellular domain introduces a non-functional receptor which can function as a membrane bound extracellular marker.
Chimeric proteins consisting of leptin and a nanobody against human TNFR1 (here nb96) will bind to cells carrying the mLR and to cells carrying the hTNFR1. Chimeric proteins with leptin mutants L86S and L86N have reduced affinity for the LR but retain their affinity for the hTNFR1.
Chimeric proteins were produced by transient transfection of Hek293T cells with expression plasmids. Supernatant was 0.45 µm filtered and serially diluted in 96-well plates for the assay. A serial dilution of purified recombinant leptin was used as a reference. 3000 to 10000 cells were plated per well and proliferation was measured by staining with XTT four or five days later. OD was measured at 450 nm. The results are shown in Figure 16, for two experiments using a different leptin construct (see Figure 17). For both constructs, a hTNFR depending growth stimulation can be seen for the mutant constructs, whereas the hTNFR expression does not affect the growth of the cells treated with wt (non-targeted) leptin. It is clear from these results that the targeting can compensate for the negative effect of the mutation.

### REFERENCES

- Benoit, P., Maguire, D., Plavec, I., Kocher, H., Tovey, M. and Meyer, F. (1993). J. Immunol., 150,707 -716
- Bourachot, B., Jouanneau, J., Giri, I., Katinka, M., Cereghini, S., and Yaniv, M. (1982). EMBO J., 1, 895-900.
- Brecht, A., Gauglitz, G. And Polster, J. (1993). Biosens. Bioelectron.. 8, 387-392.
- Coccia, E.M., Severa, M, Giacomini, E., Monneron, D., Remoli, M-E., Julkunen, I., Cella, M., Lande, R. and Uzé, G. (2004). Eur. J. Immunol. 34. 796-805.
- Conklin, D. (2004). J. Computiational Biol. 11, 1189-1200.
- Eyckerman, S., Waelput, W., Verhee, A., Broekaert, D., Vendekerckhove, J., and Tavernier, J. (1999). Eur. Cytok. Netw. 10, 549-559.
- Gaborit, N., Labouret, C, Vallaghe, J., Peyrusson, F., Bascoul-Mollevi, C. ? Crapez, E., Azria, D., Chardès, T., Poul, M.A., Mathis, G., Bazin, H., Pèlegrin, A. (2011). J. Biol. Chem. 286, 11337-11345.
- Ortiz-Sanchez, E., Helguera, G., Daniels, T.R. and Penichet, M.L. (2008). Expert Opin. Biol. Ther., 8, 609-632.
- Pellegrini, S., John, J., Shearer, M., Kerr, I.M. and Stark, G.R. (1989). Mol. Cell. Biol. 9, 4605-4012.
- Piehler, J., Roisman, L.C. and Schreiber, G. (2000). J. Biol. Chem. 275, 40425-40433.
- Lasfargues, E.Y., Coutino, W.G. and Dion, A.S. (1979). In Vitro, 15, 723-729.
- Roisman, L.C, Piehler, J., Trosset, J.Y., Scheraga, H.A. and Schreiber, G. (2001). Proc. Natl. Acad. Sci. YSA, 98, 13231-13236.
- Rossi, E.A., Goldenberg, D.M., Cardillo, T.M., Stein, R. And Chang, C.H. (2009) Blood, 114, 3964-3871.
- Southern, P.J. and Berg, P. (1982) J. Mol. Appl. Genet. 1, 327-341. Skerra, A. (2008). FEBS J., 275, 2677-2683.
- Stewart II W.E. The interferon system. Springer-Verlag, Wien, New York. 1979.
- Takabe, Y., Seiki, M., Fujisawa, J., Hoy, P., Yokata, K. and Arai, N. (1988). Mol. Cell. Biol. 8, 466-472.
- Tan, M.H., Nowak, N.J., Loor, R., Ochi, H., Sandberg, A.A., Lopez, C, Pickren, J.W., Berjian, R., Douglass, H.O. jr. and Chu, T.M. (1986) Cancer Invest. 4, 15-23.
- Uzé et al. Cell 60, 225-234 (1990).
- Uzé, G., Di Marco, S., Mouchel-Veilh, E., Monneron, D., Bandu, M.T., Horisberger, M.A., Dorques, A., Lutfalla, G., and Mogensen, K.E. (1994). J. Mol. Biol. 243, 245-257.
- Vaneycken, I., Devoogdt, N., Van Gassen, N., Vincke, C, Xavier, C, Wernery, U., Muyldermans, S., Lahoutte, T. And Caveliers, V. (2011). FASEB J. 25, 2433-2446.
- Weber et al. (1987) EMBO J. 6, 591-598.
- Wörn et al., FEBS Lett. 427, 357-361 (1998)
- Zabeau, L., Verhee, A., Catteeuw, D., Faes, L., Seeuws, S., Decruy, T., Elewaut, D., Peelman, F. And Tavernier, J. (2012). Biochem. J.. 441, 425-434.

## Claims

1. A targeting construct, comprising a mutant human interferon alpha 2 coupled to a targeting moiety, wherein the mutant human interferon alpha 2 is mutated in one or more amino acids of the region 144-154 and has a reduced affinity for its interferon receptor and a reduced biological activity of less than 70% of the biological activity of the wild type interferon, and wherein the targeting moiety comprises a variable domain of camelid heavy chain antibodies (VHH) directed to Her2, DC-STAMP, PD-L2, or CD20.

2. The targeting construct according to claim 1, wherein said mutant human interferon alpha 2 is selected from the group consisting of IFNα2 L153A, IFNα2 R149A and IFNα2 M148A.

3. A targeting construct according to any one of the above claims for use as a medicament.

4. The targeting construct according to any one of the above claims for use in treatment of cancer.

5. The targeting construct according to any one of the above claims for use in treatment of a viral disease.

6. The targeting construct according to any one of the above claims for use in treatment of diseases involving bone degradation.

7. A pharmaceutical composition, comprising a targeting construct according to any one of the claims, and a suitable excipient.

8. The targeting construct of claim 1, wherein the targeting moiety is directed to programmed death-ligand 2 (PD-L2).

9. The targeting construct according to claim 8 for use in treatment of cancer.

10. The targeting construct of claim 1, wherein the targeting moiety is directed to Her-2.

11. The targeting construct according to claim 10, wherein the mutant human interferon, alpha 2 comprises a R149A mutation.

12. The targeting construct according to any one of claims 10-11, for use in treatment of cancer.

## Patentansprüche

1. Ein Targeting-Konstrukt, umfassend ein mutiertes humanes Interferon alpha 2 gekoppelt an eine Targeting-Einheit, worin das mutierte humane Interferon alpha 2 in einer oder mehreren Aminosäuren der Region 144-154 mutiert ist und eine reduzierte Affinität für seinen Interferon-Rezeptor und eine verminderte biologische Aktivität von weniger als 70% der biologischen Aktivität des Wildtyp Interferons hat und worin die Targeting-Einheit eine variable Domäne der schweren Kette von Kamelantikörpern (VHH) gerichtet gegen Her2, DC-STAMP, PD-L2 oder CD20 umfasst.

2. Das Targeting-Konstrukt nach Anspruch 1, worin jenes mutierte humane Interferon alpha 2 ausgewählt ist aus der Gruppe bestehend aus IFNα2 L153A, IFNα2 R149A und IFNα2 M148A.

3. Ein Targeting-Konstrukt nach einem der vorherigen Ansprüche zur Verwendung als Medikament.

4. Das Targeting-Konstrukt nach einem der vorherigen Ansprüche zur Verwendung bei der Behandlung von Krebs.

5. Das Targeting-Konstrukt nach einem der vorherigen Ansprüche zur Verwendung bei der Behandlung einer viralen Erkrankung.

6. Das Targeting-Konstrukt nach einem der vorherigen Ansprüche zur Verwendung bei der Behandlung von Krankheiten, die mit Knochen-Degradation assoziiert sind.

7. Eine pharmazeutische Zusammensetzung, umfassend ein Targeting-Konstrukt nach einem der vorherigen Ansprüche und einen passenden Hilfsstoff.

8. Das Targeting-Konstrukt von Anspruch 1, worin die Targeting-Einheit gegen programmed death-ligand 2 (PD-L2) gerichtet ist.

9. Das Targeting-Konstrukt nach Anspruch 8 zur Verwendung bei der Behandlung von Krebs.

10. Das Targeting-Konstrukt von Anspruch 1, worin die Targeting-Einheit gegen Her-2 gerichtet ist.

11. Das Targeting-Konstrukt nach Anspruch 10, worin das mutierte humane Interferon alpha 2 eine R149A Mutation umfasst.

12. Das Targeting-Konstrukt nach einem der Ansprüche 10-11 zur Verwendung bei der Behandlung von Krebs.

## Revendications

1. Construction de ciblage, comprenant un interféron alpha 2 humain mutant couplé à un élément de ciblage, dans laquelle l'interféron alpha 2 humain mutant est muté dans un ou plusieurs acides aminés de la région 144 à 154 et présente une affinité réduite pour son récepteur d'interféron et une activité biologique réduite de moins de 70 % de l'activité biologique de l'interféron de type sauvage, et dans laquelle l'élément de ciblage comprend un domaine variable d'anticorps de chaîne lourde (VHH) de camélidés dirigés contre Her2, DC-STAMP, PD-L2, ou CD20.

2. Construction de ciblage selon la revendication 1, dans laquelle l'interféron alpha 2 humain mutant est choisi dans le groupe constitué de l'IFNα2 L153A, l'IFNα2 R149A et l'IFNα2 M148A.

3. Construction de ciblage selon l'une quelconque des revendications ci-dessus pour une utilisation en tant que médicament.

4. Construction de ciblage selon l'une quelconque des revendications ci-dessus pour une utilisation dans le traitement du cancer.

5. Construction de ciblage selon l'une quelconque des revendications ci-dessus pour une utilisation dans le traitement d'une maladie virale.

6. Construction de ciblage selon l'une quelconque des revendications ci-dessus pour une utilisation dans le traitement de maladies impliquant une dégradation osseuse.

7. Composition pharmaceutique, comprenant une construction de ciblage selon l'une quelconque des revendications, et un excipient approprié.

8. Construction de ciblage selon la revendication 1, dans laquelle l'élément de ciblage est dirigé contre le *programmed death-ligand* 2(PD-L2).

9. Construction de ciblage selon la revendication 8 pour une utilisation dans le traitement du cancer.

10. Construction de ciblage selon la revendication 1, dans laquelle l'élément de ciblage est dirigé contre Her-2.

11. Construction de ciblage selon la revendication 10, dans laquelle l'interféron alpha 2 humain mutant comprend une mutation R149A.

12. Construction de ciblage selon l'une quelconque des revendications 10 et 11, pour une utilisation dans le traitement du cancer.
